# NEUE EUROPÄISCHE PATENTSCHRIFT

(11) **EP 2 380 524 B2**
(45) Veröffentlichungstag und Bekanntmachung des Hinweises auf die Entscheidung über den Einspruch: **04.09.2024**
(45) Hinweis auf die Patenterteilung: 21.06.2017
(21) Anmeldenummer: 11174531.1
(22) Anmeldetag: 13.05.2009
(51) Int. Cl.: A61C 8/00, A61B 17/88, B29C 65/06, B29C 65/08, B29C 65/56, F16B 13/12

(54) **Vorrichtung und Verfahren zur Amelioration von Ausnehmungen**
Method and device for ameliorating recesses
Procédé et dispositif destinés à l'amélioration des évidements

(30) Priorität: 21.05.2008 CH 7602008
(43) Veröffentlichungstag der Anmeldung: 26.10.2011
(62) Teilanmeldung aus: 09749742.4
(73) Patentinhaber: Nexilis AG, 4410 Liestal (CH)
(72) Erfinder: Schlottig, Falko, 4414 Füllinsdorf (CH); Werner, Uwe, 8042 Uitikon (CH)
(74) Vertreter: Bremi, Tobias Hans

(56) Entgegenhaltungen:
- WO-A1-02/069817
- WO-A1-2008/034278
- WO-A1-2008/128367
- WO-A1-2009/052644
- WO-A1-2010/045751
- WO-A2-2008/034277
- US-A- 4 100 954

## Beschreibung

### TECHNISCHES GEBIET

Die vorliegende Erfindung betrifft eine Vorrichtung zur Amelioration einer Ausnehmung, insbesondere einer Ausnehmung in einem porösen oder löchrigen respektive durch die Ausnehmung freigelegte Hohlräume aufweisenden Material wie beispielsweise Holz, technisches Material, einem tierischen oder menschlichen Knochen. Des weiteren betrifft sie Verfahren zur Amelioration von Ausnehmungen in porösen oder löchrigen respektive durch die Ausnehmung freigelegte Hohlräume aufweisenden Materialien wie beispielsweise tierischem oder menschlichem Knochen, insbesondere in Kieferknochen oder Wirbelsäulenknochen.

### STAND DER TECHNIK

Insbesondere aus dem Bereich der Befestigung von Implantaten in Ausnehmungen im tierischen oder menschlichen Körper wie bspw. in Bohrungen in Knochen ist es bekannt, Implantate, welche bspw. mit einem selbstschneidenden Gewinde ausgestattet sind, unter Kraftanwendung in solche Ausnehmungen einzudrehen und anschliessend darauf zu warten, dass das Implantat in den Knochen einwächst.

Ebenfalls ist es bekannt, dass insbesondere bei Ausnehmungen, welche in besonders porösen Knochenabschnitten vorgesehen werden, die so genannte Primärstabilität, das heisst die Stabilität des Implantates in der Ausnehmung unmittelbar nach dem Eindrehen, das heisst bevor das eigentliche Einwachsen abgeschlossen ist, ungenügend sein kann.

Um solche Probleme zu lösen, wurde bereits vorgeschlagen (vgl. bspw. die EP 1 363 543) das Implantat wenigstens teilweise oder sogar vollständig aus einem durch mechanische Energie verflüssigbaren Material herzustellen. Das verflüssigbare Material kann nach Einbringen des Implantates in den Gewebeteil durch mechanische Schwingungen verflüssigt werden und so wird eine formschlüssige Verbindung zwischen Knochen und Implantat, vermittelt durch das verflüssigte und anschliessend wieder verfestigte Material, hergestellt. Nachteilig an derartigen Lösungen ist die Tatsache, dass ganz spezifische Implantate erforderlich sind, um derartige Verfahren durchführen zu können. Des Weiteren ist nachteilig, dass das verflüssigbare Material nicht genügend gezielt in die gewünschten Bereiche eingebracht werden kann und häufig bspw. in am Boden der Ausnehmungen angeordneten grossen Ausnehmungen verschwindet ohne am Ende zur eigentlichen Primärstabilisierung beizutragen. Grundsätzlich ist das Konzept, Ausnehmungen in einem menschlichen Körper unter Zuhilfenahme eines verflüssigbaren Materials, insbesondere im Dentalbereich, auszufüllen, bereits seit langem bekannt. So beschreibt die US 3,919,775 ein Verfahren zum Ausfüllen und Vorbereiten von Öffnungen unter Zuhilfenahme eines verflüssigbaren Materials, welches zunächst in die Öffnung eingepresst wird, und anschliessend unter Zuhilfenahme einer Sonotrode, das heisst einer Vorrichtung, mit welcher mechanische Energie in Form von Ultraschall eingetragen werden kann, verflüssigt wird. Das verflüssigte Material fliesst anschliessend in Kavitäten und Hohlräume, welche an die Ausnehmung angrenzen und verschliesst diese. Auch in anderen Gebieten, wo technische Materialien wie z.B. Holz, Kunststoffe, Schaumstoffe etc. bearbeitet werden, sind solche Techniken im weitesten Sinne bekannt.

Dokumente WO 2008034278 und WO 2008034277 offenbaren eine Vorrichtung zur Amelioration einer Ausnehmung, umfassend eine Sonotrode, eine Ameliorationshülse aus einem mit mechanischer Energie verflüssigbaren Material und einen Führungsstift sowie eine zylindrische Manschette mit einer zentralen Ausnehmung zur Aufnahme des Führungsstiftes. Der Führungsstift wird von der Ameliorationshülse umschlossen, wobei die Manschette bei Anlegen von mechanischer Energie relativ zum Führungsstift in Richtung zum Boden der Ausnehmung unter Verflüssigung und seitlicher und/oder longitudinaler Verdrängung des Materials der Ameliorationshülse verschiebbar ist.

### DARSTELLUNG DER ERFINDUNG

Die Erfindung ist in den Ansprüchen definiert.

Der Erfindung liegt demnach die Aufgabe zugrunde, eine Vorrichtung zur Amelioration einer Ausnehmung zur Verfügung zu stellen. Die Vorrichtung soll dabei insbesondere dazu geeignet sein, Ausnehmungen in einem porösen oder mit an die Ausnehmung grenzenden Löchern respektive Hohlräumen ausgestaltetem Material für weitere Bearbeitungen vorzubereiten. Insbesondere geht es um die Vorbereitung von Ausnehmungen respektive (Sack-)Löchern oder Durchgangsöffnungen in Holz oder holzartigen Werkstoffen, porösen Kunststoffen, oder Schaumstoffmaterial, insbesondere ein Polymerschaum, ein Verbundschaum und/oder ein Metallschaum, aber auch im tierischen oder menschlichen Knochen, so beispielsweise die Vorbereitung von derartigen Ausnehmungen zur anschliessenden Befestigung von Befestigungsmitteln oder Implantaten (ebenfalls eingeschlossen ist die Befestigung von Stiften oder Sehnen oder die Verankerung von künstlichen Gelenken wie beispielsweise Hüfte, Finger, Schulter etc.), so dass im Fall von nicht-tierischen respektive nicht-menschlichen Materialien die zusätzliche Verwendung von Klebstoffen vermieden werden kann und im Fall von Knochenmaterial eine schnelle Primärstabilisation des Implantates gewährleistet ist. Auf der anderen Seite soll die Amelioration auch dazu geeignet sein, eine derartige Ausnehmung gewissermassen zu versiegeln, wie dies beispielsweise in der Endodontie im Zusammenhang mit der Verschliessung von Wurzelkanälen interessant sein kann.

Die Lösung dieser Aufgabe wird dadurch erreicht, dass eine solche Vorrichtung, welche ein Element zur Erzeugung oder zur Einkopplung von mechanischer Energie, insbesondere von Vibrationsenergie respektive Schwingungsenergie wie zum Beispiel Ultraschall-Schwingungen aufweist, weiterhin eine zylindrische Manschette mit zylindrischer Mantelfläche mit einem Aussendurchmesser und mit einer zentralen Ausnehmung zur Aufnahme eines Führungsstiftes aufweist. Dabei ist der Führungsstift dazu vorgesehen und insbesondere in der Ausnehmung angeordnet, um vor dem Anlegen von mechanischer Energie, insbesondere von Schwingungen, im wesentlichen bis auf den Boden der Ausnehmung eingeführt zu sein (oder wenigstens im Bereich des Bodens der Ausnehmung beispielsweise in einer Führungsverjüngung gefangen), und so eine optimale Führung des Werkzeugs zu gewährleisten. Dabei wird der Führungsstift im Bereich seines dem Boden der Ausnehmung zugewandten Endes von einer Ameliorationshülse aus einem mit mechanischer Energie, insbesondere mit Schwingungsenergie wie bevorzugtermassen Ultraschall-Schwingungen verflüssigbaren Material umschlossen. Die (kreis-)zylindrische Mantelfläche der Ameliorationshülse weist im wesentlichen den gleichen Aussendurchmesser auf wie die Manschette, und der Führungsstift ist in der zentralen Ausnehmung derart verschieblich aufgenommen, dass die Manschette bei Anlegen von mechanischer Energie wie bevorzugtermassen Ultraschall-Schwingungen relativ zum Führungsstift in Richtung zum Boden der Ausnehmung unter Verflüssigung und seitlicher und/oder longitudinaler Verdrängung des Materials der Ameliorationshülse verschoben werden kann.

Der Führungsstift ist in der zentralen Ausnehmung bevorzugt so gut wie spielfrei verschieblich aufgenommen, das heißt der Außendurchmesser des Führungsstiftes entspricht im wesentlichen dem Innendurchmesser der zentralen Ausnehmung und ist gerade nur soviel geringer, dass der Führungsstift longitudinal in der Ausnehmung verschoben werden kann. Der Unterschied zwischen dem Außendurchmesser des Führungsstiftes und dem Innendurchmesser der Ausnehmung sollte entsprechend nicht größer sein als 0.001mm oder 0.01mm, obere Grenze ist z.B. bei Anwendungen im Implantatbereich normalerweise 0.1-0.5 mm oder 0.2-0.3mm.

Einer der Kernpunkte dieser Vorrichtung ist es also, einerseits einen Führungsstift vorzusehen, welcher in die Tiefe der Ausnehmung eingeschoben werden kann und die anschliessende Führung des Werkzeugs gewährleisten kann. Der Führungsstift dient also einerseits einer optimalen Positionierung des Werkzeugs in der Tiefe der Ausnehmung. Der Führungsstift dient aber andererseits auch zur Führung der Manschette, welche gewissermassen im oberen Bereich (bei Beginn des Prozesses) den Führungsstift umschliesst. Bei Beginn des Prozesses ist unterhalb der Manschette ebenfalls den Führungsstift umschliessend die Ameliorationshülse angeordnet. Nach der Erfindung verfügt die Ameliorationshülse über einen Aussendurchmesser, welcher gleich ist (oder gegebenenfalls unwesentlich geringer) als der Aussendurchmesser der Manschette. Typischerweise ist nämlich die Ausnehmung von zylindrischer Form. Dabei muss hervorgehoben werden, dass unter dem Begriff " zylindrische Form" zwar bevorzugtermassen eine kreiszylindrische Form zu verstehen ist (das heisst mit einem kreisrunden Querschnitt senkrecht zur Hauptachse), dass aber darunter auch Formen zu verstehen sind, welche einen ovalen respektive linsenförmigen oder ellipsenförmigen Querschnitt senkrecht zur Hauptachse des Werkzeugs aufweisen. Insbesondere im Bereich der Implantologie gibt es einerseits Ausnehmungen, welche einfach kreisförmig sind und durch die Verwendung eines rotierenden Bohrers hergestellt worden sind, es gibt aber auch zum Beispiel im Falle von dentalen Anwendungen ovale, linsenförmige oder ellipsenförmige Öffnungen (beispielsweise in definierter und aufbereiteter Form erzeugt durch ein Bohren und anschliessendes Raspeln), beispielsweise vorgegeben durch die Form einer Zahnwurzel. Im letzteren Fall, das heisst für ovale, linsenförmige oder ellipsenförmige Öffnungen, ist es also möglich, sowohl die Manschette als auch die Ameliorationshülse in ihrer Aussenform dieser eben dann z.B. oval-zylindrischen Ausnehmung anzupassen. Auch spezifische, beispielsweise auf Zahnwurzeln angepasste abgerundete andere Formen von Querschnitten sind für die Aussenform der Manschette und der Ameliorationshülse möglich.

Aufgrund der Tatsache, dass die Manschette auf dem Führungsstift verschieblich im Rahmen der Vorrichtung angeordnet ist, kann nun nach Anlegen der mechanischen Energie, z.B. der Ultraschall-Schwingungen, und entsprechender Verflüssigung des Materials der Ameliorationshülse das Material der Letzteren sukzessive von oben nach unten in die an die Ausnehmung grenzenden porösen Bereiche des die Ausnehmung bildenden Materials eingebracht werden. So werden ganz gezielt die Hohlräume, welche eine anschliessende Befestigung einer Schraube oder eines Implantats in der Ausnehmung schmälern könnten, im entscheidenden Bereich insbesondere unmittelbar am Umfang der Ausnehmung mit dem Material der Ameliorationshülse gefüllt, was eine enorme Erhöhung der Primärstabilisation nach sich zieht. Es ist aber nicht notwendigerweise zwingend, dass anschliessend überhaupt ein weiteres Element in eine solche ameliorierte Ausnehmung eingebracht wird, es ist auch möglich, durch das vorgeschlagene Verfahren respektive die vorgeschlagene Vorrichtung die Ausnehmung gewissermassen nur ganz gezielt in ihrem Umfangsbereich gewissermassen zu versiegeln (vergleiche die oben erwähnten Anwendungen im Zusammenhang mit der Endodontie).

Die Erfindung ist dadurch gekennzeichnet, dass der Aussendurchmesser von Manschette und/oder von Ameliorationshülse im wesentlichen gleich sind, oder Letzterer nur unwesentlich geringer, und dass des weiteren dieser Aussendurchmesser im wesentlichen gleich ist wie der Innendurchmesser der zu ameliorierenden Ausnehmung. Für den Fall eines nicht kreiszylindrischen Querschnittes ist dies so zu verstehen, dass der im wesentlichen gleiche Aussen-Querschnitt bei Manschette und/oder Ameliorationshülse vorliegt und die beiden Elemente relativ zueinander so angeordnet sind, dass ein im wesentlichen glatter das heisst stufenloser Übergang vorliegt. So ist bereits zu Beginn des Prozesses der distale Bereich (das heisst der dem Boden der Ausnehmung zugewandter Bereich) des Werkzeugs sowie auch der gesamte in der Ausnehmung angeordnete dahinterliegende Bereich des Werkzeugs optimal positioniert und geführt. Typischerweise verfügt die Ameliorationshülse über eine Höhe (entlang der Achse der Ausnehmung), welche geringer oder gleich ist wie die Tiefe der Ausnehmung, das heisst beim Beginn des Prozesses verschwindet die Ameliorationshülse vollständig in der Tiefe der Ausnehmung und auch die Manschette dringt teilweise in den oberen Bereich der Ausnehmung bereits ein, oder die Ameliorationshülse schliesst (insbesondere bei der Anwendung in Holz oder anderen porösen Werkstoffen) bündig mit der Oberkante des Materials ab. Typischerweise dringt die Manschette bereits 5-50% in die Ausnehmung ein, bevor der Ultraschall angelegt wird.

Es ist auch möglich, dass der Außendurchmesser der Manschette etwas größer ist als der Innendurchmesser der zu ameliorierenden Ausnehmung (beispielsweise 0.5-2 mm grösser). In diesem Fall wird beim Einführen der Vorrichtung die zu ameliorierende Ausnehmung durch die Vorrichtung noch etwas ausgeweitet und/oder in die gewünschte Form gebracht. Insbesondere bei technischen Materialien kann dies von Vorteil sein.

Eine weitere bevorzugte Ausführungsform der vorgeschlagene Vorrichtung ist dadurch gekennzeichnet, dass die zentrale Ausnehmung eine kreiszylindrische Ausnehmung ist, welche koaxial zur zylindrischen Mantelfläche angeordnet ist, dass die Ameliorationshülse eine kreiszylindrische Ausnehmung zur Aufnahme des Führungsstifts aufweist, und dass der Führungsstift eine kreiszylindrische Aussenfläche aufweist, wobei die Innendurchmesser der genannten Ausnehmungen im wesentlichen gleich sind wie der Aussendurchmesser des Führungsstiftes.

Alternativ sind aber auch andere Querschnittsform in der zentralen Ausnehmung in der Manschette möglich. So ist es beispielsweise möglich, dass die zentrale Ausnehmung eine (gleichschenklige) Dreiecksform, eine quadratische Form, oder generell eine Mehreckform (vorzugsweise mit gleich langen Seiten) aufweist, wobei die Spitzen einer solchen Querschnittsform auch bis zum Aussendurchmesser der Manschette reichen können. So kann beispielsweise gezielt in bestimmte Richtungen mehr Material vorgesehen werden. Die Querschnitts-Formen können auch runde verallgemeinerte Formen sein, sie können konkav oder konvex ausgestaltet sein. Entsprechend ist selbstverständlich dann die Querschnittsform des Führungsstiftes analog ausgebildet, wobei generell gilt, dass zwischen den Führungsstift und der Manschette ein möglichst geringer Spalt ausgebildet sein soll, damit in den Spalt zwischen diesen beiden Materialien kein verflüssigtes Material eindringen kann. Es ist aber möglich, in diesem Spalt ganz gezielt gewissermassen Kanäle vorzusehen, die ein gezieltes Abfliessen von Material bei zu hohem Druck (beispielsweise wenn keine Hohlräume zur Verdrängung des Materials vorliegen) ermöglichen. Solche Kanäle können beispielsweise das verflüssigtes Material nach oben, das heisst in Richtung aus der Ausnehmung hinaus, abführen.

Um eine optimale Verdrängung des Materials der Ameliorationshülse nicht nur nach unten sondern radial nach aussen in die Umfangsbereich mit der Ausnehmung zu verteilen, kann es von Vorteil sein, wenn die Manschette an ihrem distalen Ende eine zu diesem hin zulaufende bevorzugt umlaufende distale Kante aufweist, wobei vorzugsweise diese Kante gerade und damit konisch oder gekrümmt, insbesondere konkav oder konvex, ausgebildet ist. Diese zulaufende Kante führt dazu, dass das Material nicht nur eine Verdrängungskomponente in Richtung des Bodens der Ausnehmung erfährt, sondern auch eine radiale Komponente. Über den Neigungswinkel dieser zulaufenden Kante kann u.a. eingestellt werden, wie stark das Material in radialer Richtung (das heisst senkrecht zur zentralen Achse der Ausnehmung respektive senkrecht zur Hauptachse des Werkzeugs) respektive longitudinaler Richtung (das heisst in Richtung zum Boden der Ausnehmung und damit parallel zur zentralen Achse der Ausnehmung respektive parallel zur Hauptachse des Werkzeugs) verdrängt werden kann. Soll beispielsweise möglichst wenig zum Boden der Ausnehmung verdrängt werden (weil beispielsweise in diesem Bereich bekanntermassen grosse Hohlräume vorhanden sind, deren Ausfüllung weder erwünscht noch notwendig ist), so kann ein sehr spitzer Winkel gewählt werden (beispielsweise < 45°, wobei der Winkel definiert ist als der Winkel zwischen der zentralen Hauptachse der Manschette und der Neigungsfläche der Kante) so dass eine grosse radiale Komponente entsteht. Soll auf der anderen Seite möglichst viel Material zum Boden der Ausnehmung verteilt werden, so kann ein Winkel grösser als 45° gewählt werden. Auf jeden Fall sollte dieser Winkel bevorzugtermassen kleiner oder gleich 90° sein, bei grösseren Winkeln wird das Material (was aber ebenfalls gewünscht sein kann) ausschliesslich zum Boden geschoben.

Die Manschette kann am distalen Ende auch gestuft ausgebildet sein. Da die Manschette vorzugsweise direkt an die Sonotrode angeschlossen ist, ist es von Vorteil, wenn die Wandstärke der Manschette nicht zu gering ist. Typischerweise sollte die Wandstärke der Manschette für typische Anwendungen im medizinischen Bereich im Bereich von 0.3-1 mm, vorzugsweise im Bereich von 0.5-0.8 mm sein. Auf der anderen Seite kann es für bestimmte Anwendungen von Vorteil sein, wenn die Ameliorationshülse eine geringere Wandstärke aufweist, damit nicht zu viel Material eingetragen wird. Die Ameliorationshülse hat bevorzugtermassen eine Wandstärke im Bereich von 0.1-1 mm, und kann beispielsweise auch nur halb so dick sein wie die Wandstärke der Manschette. Um diesen Verhältnissen Rechnung zu tragen, ist es möglich, die Ameliorationshülse auf deren Innenseite hinter deren distalem Ende auszunehmen, vorzugsweise umlaufend auszunehmen. Entsprechend resultiert eine geringere Wandstärke der Ameliorationshülse, und es entsteht zwischen Führungsstift und Ameliorationshülse ein Hohlraum.

Es ist bei solchen dünnen Ameliorationshülsen dann eben vorzugsweise auch möglich, die Manschette an deren distalem Ende gestuft auszubilden, wobei am distalen Ende der Manschette ein zylindrischer Bereich mit geringerem Außendurchmesser vorgesehen ist, welcher in diesen Hohlraum der Ameliorationshülse respektive den Zwischenraum zwischen Ameliorationshülse und Führungsstift eingreift und umlaufend von der Ameliorationshülse mit dünnerer Wandstärke umschlossen ist. Dieser Bereich mit geringerem Außendurchmesser geht bevorzugtermassen in einer geneigten Flanke über in einen Bereich mit dem eigentlichen Außendurchmesser der Manschette. In diesem geneigten Bereich erfolgt die Verflüssigung der Ameliorationshülse und der Transport des verflüssigten Materials radial nach außen.

Erfindungsgemäss kann der Führungsstift bei der vorgeschlagenen Vorrichtung bis zu einer Anschlagsposition in die Manschette eingeschoben werden, damit eine definierte Endposition für das mit der Vorrichtung geplante Verfahren vorhanden ist. Dabei schliesst bei dieser Anschlagsposition der Führungsstift bündig mit dem distalen Ende der Manschette ab.

Zur guten Positionierung des Führungsstiftes im Bereich des Bodens der Ausnehmung kann es von Vorteil sein, wenn der Führungsstift an seinem distalen Ende kegelförmig zulaufend und bevorzugt mit einer scharfen Spitze ausgebildet ist oder es kann von Vorteil sein, wenn der Führungsstift an diesem Ende abgerundet ausgestaltet ist. Die Rundung respektive generell die Gestaltung der Spitze kann dabei angepasst sein an den typischerweise zur Vorbereitung der Ausnehmung verwendeten Bohrer. Wird beispielsweise ein Bohrer verwendet, welcher einen Versatz oder eine Verjüngung zur Führung an der Spitze aufweist, und der Führungsstift weist vorzugsweise einen an den Durchmesser dieser Verjüngung angepassten Aussendurchmesser auf.

Die Vorrichtung ist bevorzugtermassen dadurch gekennzeichnet, dass der Aussendurchmesser der Manschette im Bereich von 1-50mm oder sogar 1-80 mm, bevorzugt im Bereich von 2-10 mm beträgt. Weiterhin wird bevorzugt, dass der Aussendurchmesser des Führungsstiftes 0.1 - 20 mm, bevorzugt 0.5-10 mm, insbesondere bevorzugt 1- 5 mm, geringer ist, und dass die Ameliorationshülse eine Dicke aufweist, so dass deren Aussendurchmesser gleich ist wie der Aussendurchmesser der Manschette.

Typischerweise erzeugt das Element (die eigentliche Sonotrode) mechanische Energie in Form von Schwingungsenergie mit einer Frequenz im Bereich von 1 kHz-10 GHz. Bevorzugt wird dabei, dass die Schwingung Energie als Ultraschall-Schwingungen im Frequenzbereich von 10 kHz bis 10 GHz eingetragen wird. Bevorzugt wird ein Frequenzbereich von 10 kHz - 100 MHz, insbesondere bevorzugt ein Bereich von 40 kHz- 100 MHz. Normalerweise werden Ultraschall-Schwingungen im Bereich von 20-150 kHz, bevorzugtermassen im Bereich von 25-50 kHz eingesetzt. Diese Schwingungen können in longitudinaler (das heisst entlang der Achse der Ausnehmung), transversaler (das heisst radial zur Achse der Ausnehmung) oder rotativer (das heisst um die Achse der Ausnehmung) Richtung, oder einer Kombination resp. Linearkombination dieser Richtungen auf Manschette und/oder Führungsstift und damit mittelbar auf die Ameliorationshülse übertragen werden. Vorzugsweise werden die Schwingungen in longitudinaler Richtung angelegt, wobei dann, wenn z.B. am distalen Ende der Manschette eine geneigte Flanke vorhanden ist, diese longitudinale Schwingung einen gezielten Transport des verflüssigten Materials in radialer Richtung ermöglicht. Generell bevorzugtermassen ist die Manschette an der Sonotrode befestigt und der Führungsstift kann in dieser verschoben werden. Alternativ ist es möglich, dass der Führungsstift an der Sonotrode befestigt ist und die Manschette verschoben werden kann.

Eine bevorzugte Ausführungsform der Vorrichtung ist dadurch gekennzeichnet, dass es sich bei der Ausnehmung um eine Ausnehmung in einem wenigstens teilweise porösen (menschlichen) Knochenabschnitt handelt, insbesondere in einem Kieferknochen oder einem Wirbelsäulenknochen, und dass bevorzugtermassen die Ausnehmung wenigstens teilweise durch eine Vorbohrung erzeugt ist.

Bevorzugtermassen besteht die Ameliorationshülse aus einem durch die genannte mechanische Energie, insbesondere durch Schwingungsenergie verflüssigbaren Material ausgewählt aus der Gruppe: thermoplastischer biokompatibler Polymere wie beispielsweise Polyolefine (z. B. PP, LDPE, HDPE, UHMWPE), Polyoxymethylen (POM), Polyaryletherketone (z.B. PAEK, PEEK, PEKK), Polycarbonate (PC), Polyacrylate (z.B. PMMA), Polyamide (PA), Polyester (z.B. PET, PBT), Polysulfone und Polyethersulfone (z.B. PSU, PES) und/oder biodegradierbare respektive resorbierbare Polymere wie beispielsweise Poly-(L-lactide) (PLLA), poly-(D,L-lactide) (PDLLA), und derer Stereocopolymere mit variierendem Verhältnis des L und D,L Anteils, Polyglycolide (PGA) und Copolymere wie Polyglycolide-cotrimethylencarbonat (PGA-co-TMC), Poly-(D,L-lactide-co-glycolide) (PDLLA-co-PGA), und Poly-(L-lactide-co-glycolide) (PLLA-co-PGA), Poly(e-caprolactone), Polydioxanone, Trimethylene carbonate (TMC), Polyorthoester (POE) und andere Polyanhydride, resorbierbare Polymere welche aus natürlich Rohstoffen hergestellt werden wie beispielsweise modifizierte Polysaccharide (Zellulose, Chitin, Dextran, Stärke), oder eine Kombination respektive einer Mischung dieser Materialien. Grundsätzlich können in diesem Material oder als Schicht auf diesem Material aufgetragen auch eine oder mehrere pharmazeutische Wirksubstanzen vorgesehen werden, dies beispielsweise generell Wirksubstanzen zur Verbesserung der Einheilung, wie beispielsweise zur Förderung des Knochenwachstums, zur Verhinderung von Entzündungen etc.. Das Material kann dabei spezifisch dazu ausgelegt sein, diese pharmazeutischen Wirksubstanzen kontrolliert, das heisst über einen kontrollierten Zeitraum in einer kontrollierten Dosis, freizusetzen.

Die Ameliorationshülse kann materialseitig geschlossen, aber auch durchbrochen in jeweils verschiedenen Formen wie beispielsweise gelocht oder geschlitzt ausgeführt sein, um die einzubringende Materialmenge, wenn erforderlich, den anatomischen Gegebenheiten respektive der Materialdichte respektive spezifisch der Knochendichte/qualität und damit den an die Ausnehmung angrenzenden Hohlräumen anpassen zu können

Ebenso betrifft die vorliegende Erfindung ein nicht in vivo Verfahren zum Betrieb einer Vorrichtung, wie sie oben beschrieben worden ist. Das Verfahren ist bevorzugtermassen dadurch gekennzeichnet, dass die Vorrichtung mit aufgesetztem Führungsstift und aufgesetzter Ameliorationshülse in eine, ggf. zuvor ausgebohrte (und ev. zusätzlich zu einer z.B. ovalen Form ausgeraspelte), Ausnehmung mit einem Innendurchmesser, welcher im wesentlichen dem Aussendurchmesser von Manschette respektive Ameliorationshülse entspricht (vorzugsweise beträgt der Abstand zwischen Ausnehmungen und Manschette respektive Ameliorationshülse nicht mehr als 1 mm, vorzugsweise nicht mehr als 0.5 mm, insbesondere vorzugsweise nicht mehr als 0.1 mm), derart eingeschoben wird, bis vorzugsweise der Führungsstift auf dem Boden der Ausnehmung anschlägt, und/oder in eine am Boden der Ausnehmung angeordnete Führungsverjüngung eingreift, und anschliessend unter gleichzeitiger Verflüssigung der Ameliorationshülse durch angelegte mechanische Energie wie beispielsweise durch angelegten Ultraschall und Hineinschieben der Manschette mit deren distalem Ende in die Ausnehmung hinein verflüssigtes Material in insbesondere laterale Hohlräume angrenzend an die Ausnehmung eingetragen wird. Dies gilt gleichermassen für Anwendungen in Materialien wie beispielsweise Holz oder Schaumstoffmaterial, insbesondere Polymerschaum, Verbundschaum und/oder Metallschaum etc..

Zu guter letzt betrifft die vorliegende Erfindung ein nicht in vivo Verfahren zur Amelioration einer Ausnehmung in einem porösen Material wie beispielsweise Holz, unter Verwendung einer Vorrichtung, wie sie oben geschildert wurde. Das nicht in vivo Verfahren ist bevorzugtermassen dadurch gekennzeichnet, dass die Vorrichtung mit aufgesetztem Führungsstift und aufgesetzter Ameliorationshülse in die ggf. zuvor ausgebohrte (und gegebenenfalls zusätzlich in einem zusätzlichen oder gleichzeitigen Arbeitsschritt in eine nicht-kreisrunde Querschnittsform gebrachte) Ausnehmung mit einem Innendurchmesser, welche im wesentlichen dem Aussendurchmesser von Manschette respektive Ameliorationshülse entspricht, derart eingeschoben wird, bis vorzugsweise der Führungsstift auf dem Boden der Ausnehmung anschlägt, und/oder in einer am Boden der Ausnehmung angeordnete Führungsverjüngung der Ausnehmung eingreift, und anschliessend unter gleichzeitiger Verflüssigung der Ameliorationshülse durch angelegte mechanische Energie, bevorzugt durch angelegten Ultraschall und hineinschieben der Manschette mit deren distalem Ende in die Ausnehmung hinein verflüssigtes Material in insbesondere laterale Hohlräume angrenzend an die Ausnehmung eingetragen wird, wobei bevorzugtermassen anschliessend in die ameliorierte Ausnehmung ein Befestigungsmittel, ein Gelenk, ein Gelenkabschnitt, oder eine Schraube etc., ggf. teilweise selbstschneidend eingedreht wird.

Weitere bevorzugte Ausführungsformen der Erfindung sind in den abhängigen Ansprüchen beschrieben.

### KURZE ERLÄUTERUNG DER FIGUREN

Die Erfindung soll nachfolgend anhand von Ausführungsbeispielen im Zusammenhang mit der Zeichnung näher erläutert werden. Es zeigen:
- Fig. 1: Schnittdarstellung eines Bereiches eine Lücke in der Zahnreihe, in welchem ein Implantat gesetzt werden soll;
- Fig. 2: Schnittdarstellung nach Fig. 1, Phase des Bohrens / Ausbohrens der Aufnahmeausnehmung für das Implantat;
- Fig. 3: Schnittdarstellung nach Fig. 1, Phase der Auswahl und des Aufsetzens/der Aufnahme der Ameliorationshülse mit Führungsstift auf Sonotrode resp. In Manschette;
- Fig. 4: Schnittdarstellung nach Fig. 1, Phase des Einsetzens der Ameliorationshülse in die Bohrung, Situation unmittelbar vor dem Beginn der Anregung mit der Sonotrode;
- Fig. 5: Schnittdarstellung nach Fig. 1, Phase der Anregung mit der Sonotrode (Zwischenstand, mit Detailausschnitt der Spitzenregion);
- Fig. 6: Schnittdarstellung nach Fig. 1, Phase nach der Anregung mit der Sonotrode (Endzustand, mit Detailausschnitt der Spitzenregion);
- Fig. 7: Schnittdarstellung nach Fig. 1, Zustand nach der Anregung mit der Sonotrode und nach deren Entnahme aus der ameliorierten Bohrung (mit Detailausschnitt der Spitzenregion);
- Fig. 8: Einschrauben des Implantats, manuell mit Abstützung durch Finger;
- Fig. 9: eingesetztes Implantat;
- Fig. 10: schematische Ansicht eines in eine analog ameliorierte Öffnung in einem Wirbelsäulenbereich eingesetztes Implantat/Schraube;
- Fig. 11a) - e): unterschiedliche Querschnittsgestaltungen des Führungsstiftes in Schnittdarstellungen senkrecht zur zentralen Achse der Manschette resp. der Ameliorationshülse auf einer Höhe, in welcher die Ameliorationshülse geschnitten wird;
- Fig. 12: einen axialen Schnitt durch eine Vorrichtung mit einer Ameliorationshülse mit dünner Wandstärke;
- Fig. 13: einen axialen Schnitt durch eine Vorrichtung die in einer Ausnehmung eingeführt ist, welche einen geringeren Innendurchmesser aufweist als der Außendurchmesser der Manschette.

### WEGE ZUR AUSFÜHRUNG DER ERFINDUNG

Die oben im allgemeinen beschriebene Erfindung soll nun im Zusammenhang mit den Figuren im Detail weiter erläutert werden. Die nun anfolgende Beschreibung soll zur Stützung der Ansprüche hinzugezogen werden, nicht aber zu deren Einschränkung verwendet werden.

Im Sinne eines ersten Ausführungsbeispiels der vorliegenden Erfindung soll beschrieben werden, wie eine Vorrichtung, wie sie oben beschrieben wurde, zur Befestigung eines Dentalimplantates eingesetzt werden kann. Das analoge Verfahren unter Verwendung einer analogen Vorrichtung kann aber, wie bereits eingangs erläutert, gleichermassen für Ausnehmungen in beispielsweise Holz oder anderen porösen Werkstoffen wie Schaumstoffmaterial, insbesondere Polymerschaum, Verbundschaum und/oder Metallschaum etc. eingesetzt werden.

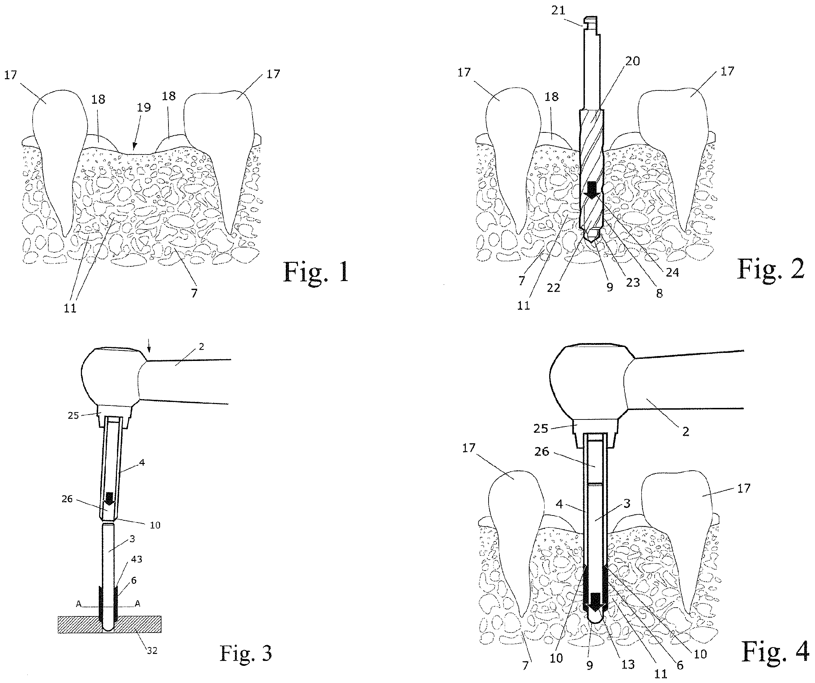

So ist in Figur 1 ein Schnitt durch einen Kieferknochen 7 angegeben, bei welchem zwischen zwei Zähnen 17 eine Lücke 19 angeordnet ist, bei welcher auch das Zahnfleisch 18 unterbrochen ist. Der Knochen weist wie üblich insbesondere unterhalb der Kortikalis (im so genannten trabekulären respektive spongösen-Bereich) eine poröse Struktur mit Hohlräumen resp. Kavitäten 11 auf. In eine solche Lücke 19 soll ein Implantat eingesetzt werden. Typischerweise weist ein derartiger Knochen im trabekulären respektive spongösen Bereich eine Porosität im Bereich von 30-90% auf, das heisst die eigentlich tragende und damit für eine Halterung geeignete Struktur macht unter Umständen nur einen geringen Bruchteil des Volumens aus.

Entsprechend wird so vorgegangen, dass in einem ersten Schritt, der schematisch in Figur 2 dargestellt ist, unter Zuhilfenahme eines Bohrers 20 die eigentliche Ausnehmung für das Implantat vorbereitet wird. Der in diesem Ausführungsbeispiel verwendete Bohrer 20 verfügt auf der einen Seite über einen Befestigungsflansch für den Antrieb (Antrieb nicht dargestellt), und auf der anderen Seite über den eigentlichen das Bohrloch erzeugenden Bohrabschnitt mit den bekannten Zügen 22 des eigentlichen Bohrers. Dieser spezifische Bohrer verfügt über eine Verjüngung an der Spitze, das heisst über einen Bereich mit einem geringeren Durchmesser als der eigentliche Bohrbereich, diese Verjüngung führt in der resultierenden Ausnehmung zu einem verjüngten Bodenbereich, in welchem der Durchmesser etwas geringer ist als im weiter oberhalb liegenden Bereich der Ausnehmung. Es handelt sich beispielsweise um einen Bohrer, wie er in der WO 2004/080325 beschrieben wird.

Der Bohrer wird, wie dies durch den Pfeil 24 angegeben ist, unter Drehung entlang seiner Achse in den Knochen 7 eingetrieben und es bildet sich dabei die eigentliche Ausnehmung 8. Aufgrund der Ausbildung dieser Ausnehmung 8 in porösen Knochen werden im peripheren Bereich dieser Ausnehmung Hohlräume 11, welche an die Ausnehmung angrenzen, gewissermassen freigelegt und geöffnet.

In einem nächsten Schritt wird nun diese Ausnehmung 8 so genannt amelioriert, das heisst entweder für die Befestigung eines Implantates in einer solchen Ausnehmung vorbereitet oder einfach nur bezüglich der offen liegenden Hohlräume 11 versiegelt.

Zu diesem Zweck wird die vorgeschlagene Vorrichtung (vgl. Figur 3) in diesem Fall so präpariert, indem bei der Vorrichtung 1, welche über einen Handgriff mit einem Element zur Erzeugung von Ultraschall versehen ist, und an welchem über eine obere Befestigung 25 eine Manschette 4 (zylindrischer Rohrabschnitt mit zentraler Ausnehmung 26) angeordnet ist, ein Führungsstift 3, welcher an seinem unteren Ende über eine Ameliorationshülse 6, welche den Führungsstift umlaufend umschliesst, verfügt, in die genannte Ausnehmung 26 der Manschette 4 eingeschoben wird. Bspw. ist es möglich, dass solche Führungsstifte 3 mit Ameliorationshülsen 6 in einer Halterung 32 in unterschiedlichen Ausbildungen (bspw. unterschiedliche Dicken von Ameliorationshülsen) vorgelegt werden. Das Material der Ameliorationshülse ist dabei so ausgewählt, dass es durch Anlegen von mechanischer Energie, insbesondere von Ultraschall-Schwingungen verflüssigt werden kann. Die Manschette 4 verfügt an ihrem distalen Ende (das heisst an dem Handgriff 2 abgewandten Ende) über eine zur Spitze hin zulaufende umlaufende Kante 10. Diese konische Spitze 10 greift bei eingeschobenen Führungsstift 3 in einen hinterschnittenen oberen Bereich der Ameliorationshülse (mit dem Bezugszeichen 43 bezeichnet) ein.

Im Zusammenhang mit Figur 3 sei darauf hingewiesen, dass entweder, wie hier dargestellt, Führungsstift und Ameliorationshülse eine Einheit bilden können, es ist aber auch möglich, dass Manschette, Führungsstab und Ameliorationshülse eine Einheit für den Einmalgebrauch darstellen. Ebenfalls möglich ist es, dass Manschette und Ameliorationshülse, oder andere Kombinationen von Elementen, eine Einheit für den Einmalgebrauch bilden. Diese Einheiten können in einer sterilen Packung vorgelegt werden.

Als Material für die Ameliorationshülse kann beispielsweise eine resorbierbares polymeres Material des Typs Resomer wie erhältlich von Boehringer Ingelheim (DE) eingesetzt werden. Es kann dabei auf Basis von Homopolymeren aus Milchsäure (Polylactide) oder auf Basis von Copolymeren aus Milch- und Glycolsäure aufgebaut sein, und es kann zudem vorzugsweise dazu ausgelegt sein, eine kontrollierte Freisetzung pharmazeutischer Wirksubstanzen (beispielsweise generell Wirksubstanzen zur Verbesserung der Einheilung, wie beispielsweise zur Förderung des Knochenwachstums, zur Verhinderung von Entzündungen etc.), welche in diesem Material eingebracht oder auf dieses aufgetragen sind, zu gewährleisten.

Im vorliegenden Fall, wo der Bohrer wie oben beschrieben einen verjüngten Bodenbereich 9 in der Ausnehmung erzeugt hat, ist die Ameliorationshülse 6 vorzugsweise nicht ganz an der Spitze des Führungsspitzes angeordnet sondern etwas nach hinten versetzt, und der Führungsstift verfügt über einen Aussendurchmesser welcher im Wesentlichen dem Durchmesser der Verjüngung 9 im Bodenbereich entspricht. Die so vorbereitete Vorrichtung 1 wird anschliessend, wie dies in Figur 4 dargestellt ist, in die vorgebohrte Ausnehmung 8 eingeschoben. Der Aussendurchmesser der Manschette 4 ist im Wesentlichen identisch zum Aussendruchmesser D der Ameliorationshülse, und dieser Aussendurchmesser ist im Wesentlichen gleich oder höchstens unwesentlich geringer als der Innendurchmesser der durch den Bohrer erzeugten Ausnehmung. Wie weiter oben erläutert, ist es auch möglich, dass der Außendurchmesser der Manschette etwas größer ist als der Innendurchmesser der Ausnehmung, so dass bei der Führung des Prozesses eine zusätzliche Aufweitung der Ausnehmung erfolgt.

Wie dies aus Figur 4 ersichtlich wird kann entsprechend die Einheit aus Führungsstift 3,

Ameliorationshülse 6 und Manschette 4 im Wesentlichen vollständig in die Ausnehmung eingeschoben werden und insbesondere dringt auch die vorderste Spitze des Führungsstiftes in den verjüngten Bodenbereich 9 ein und auch in radialer Richtung füllt der vordere Bereich der Vorrichtung die Ausnehmungen vollständig aus. So ergibt sich automatisch eine optimale Zentrierung respektive Positionierung der Vorrichtung in der Ausnehmung. Die Vorrichtung wird also ohne Anlegen von Ultraschall entsprechend der Verschiebungsrichtung 13 in die Ausnehmung im wesentlichen vollständig eingeschoben.

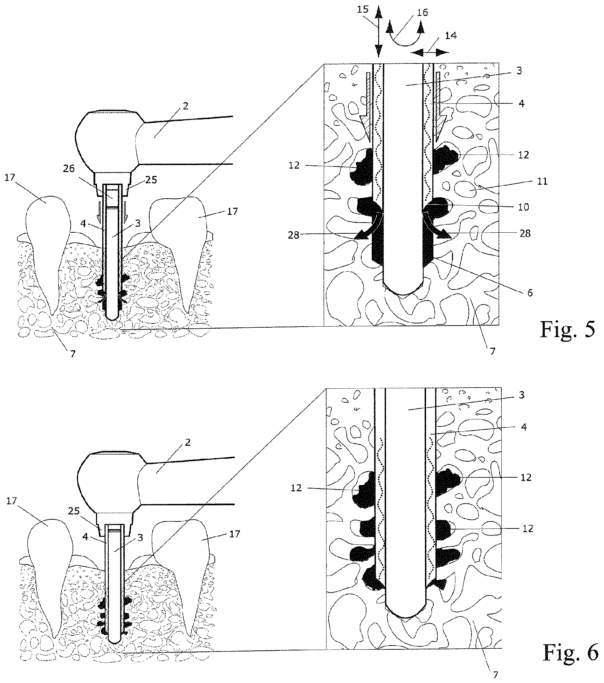

Erst jetzt wird, wie dies in Figur 5 dargestellt wird, der Ultraschall eingeschaltet. Als der Vorrichtung zur Erzeugung von Ultraschall kann beispielsweise eine Vorrichtung der Firma Branson Ultrasonics SA, CH, vom Typ E-150 mit einer Arbeitsfrequenz von 20 kHz und einer Amplitude von 40 µm an der Spitze der Sonotrode mit einer Energie von 150 W eingesetzt werden. Die mechanische Energie kann Ihre Anregung über die Manschette eintragen, dies ist bevorzugt, es ist aber auch möglich, die Energie über den Führungsstift einzutragen, oder sowohl über die Manschette wie auch über den Führungsstift. Dabei verflüssigt sich das Material der Ameliorationshülse sukzessive, und gleichzeitig wird nun durch Druck auf den Handgriff 2 die Manschette 4 immer weiter auf den Führungsstift aufgeschoben. Infolge der abgeschrägten Kante 10 der Manschette 4 wird das verflüssigte Material nun ganz gezielt über den Umfang sukzessive von oben nach unten verteilt in die durch die Ausnehmung freigelegten Hohlräume 11 eingetragen. Das verflüssigte Material wird also ganz gezielt in radialer Richtung über den Umfang verteilt in diese Hohlräume 11, soweit sie durch das Bohren freigelegt wurden, eingetragen, und es findet nicht die meist nachteilige Verschiebung nur nach unten auf den Boden der Ausnehmung statt (was aufgrund der Tatsache, dass Knochenmaterial sehr häufig mitzunehmender Tiefe immer poröser wird in der Regel genau problematisch ist). Des Weiteren findet nicht eigentlich eine Auskleidung der Ausnehmung durch das verflüssigte Material statt sondern es werden nur die unerwünschten an die Ausnehmung angrenzenden Hohlräume gewissermassen ausgefüllt. Jene Bereiche, in welchen der Knochen bereits eine saubere Begrenzung der Ausnehmung bildet, bleiben von verflüssigbarem Material im Wesentlichen unbedeckt. Durch die Einstellung des Winkels der Kante 10 kann die radiale Komponente, wie sie schematisch durch die Pfeile 28 dargestellt ist, eingestellt werden.

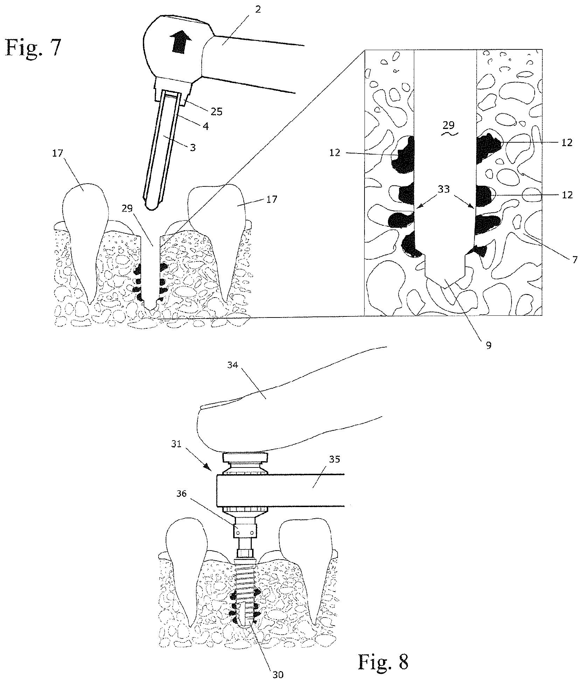

In Figur 6 ist nun nicht mehr der in Figur 5 dargestellte Zwischenzustand abgebildet sondern der eigentliche nicht-erfindungsgemässe Endzustand, bei welchem die Manschette bis zu einem Anschlag auf den Führungsstift 3 aufgeschoben ist und sämtliches Material der Ameliorationshülse in die radial angrenzenden Hohlräume verteilt ist. Insbesondere aus der Detailansicht kann ersehen werden, dass das Material der Ameliorationshülse in der verdrängten Form 12 ausschliesslich in den unerwünschten radial angrenzenden Kavitäten angeordnet ist und nicht einfach nur nach unten transportiert wird. Genau dort, wo das Implantat seine Primärstabilisierung bezieht, namentlich auf der umlaufenden Zylinderfläche der Ausnehmung, wird dadurch eine wesentlich erhöhte Angriffsfläche für eine Verankerung des Implantates zur Verfügung gestellt, und gleichzeitig so wenig Fremdmaterial wie möglich in den Knochen eingebracht. Die Manschette muss nicht zwingend bis zum Anschlag aufgeschoben werden, und es ist auch möglich, den Prozess etwas vorher abzubrechen, wenn Material der Ameliorationshülse am Boden nicht in radialer Richtung verdrängt verbleiben soll.
Anschliessend kann das Werkzeug, wie dies in Figur 7 dargestellt ist, entweder bei eingeschaltetem oder bei ausgeschaltetem Ultraschall, aus der Ausnehmung wieder entfernt werden und die Ausnehmung ist nun im Sinne der Erfindung amelioriert, wie dies durch das Bezugszeichen 29 dargestellt ist. Dies bedeutet, dass die Umfangsfläche 33 der ameliorierten Ausnehmung 29 wenigstens überall dort, wo Hohlräume angrenzend waren, durch das verflüssigbare Material begrenzt ist. Das verflüssigbare Material erstarrt selbstverständlich wieder, nachdem der Ultraschall-Eintrag abgeschaltet worden ist.

Entweder kann die so ameliorierte Ausnehmung in gewissermassen versiegeltem Zustand belassen werden, sofern dies das Ziel der Operation ist, oder es kann, wie dies in der Regel der Fall sein wird, nun das eigentliche Implantat in der Ausnehmung befestigt werden. Dieser Schritt ist in Figur 8 dargestellt für eine Situation, in welcher eine Vorrichtung 31 zum Eindrehen des Implantats mit einem Finger 34 nach unten gedrückt und mit einem -Drehmomentschlüssel 35 angetrieben wird. Die eigentliche Montagevorrichtung 36 wird auf der Innenseite des Implantates 30 an diesem befestigt und anschliessend an die Montage wieder entfernt. Das Implantat kann auch mit dem Handstück (maschinell) eingebracht werden. Dann kann normalerweise die Sonotrode nicht auch noch angesetzt werden.

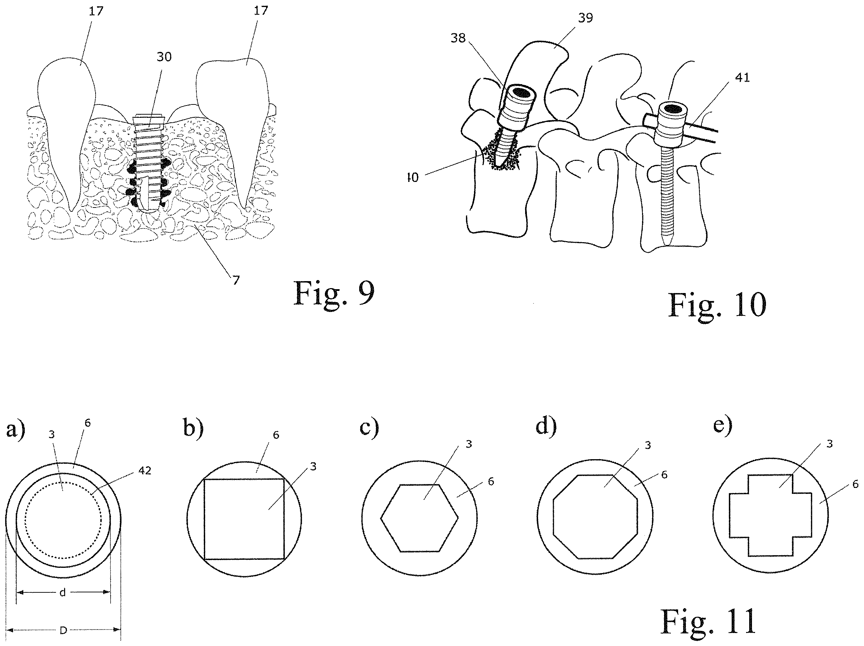

In Figur 9 ist das entsprechend eingesetzte Implantat dargestellt, auf ein solches Implantat 30 kann nun bspw. die eigentliche Zahnprothese aufgesetzt werden.

Wie eingangs erläutert, kann das vorgeschlagene Verfahren generell bei einem menschlichen Gewebeteil oder einem menschlichen Knochen, gleichermassen bei einem tierischen Knochen oder generell bei einem porösen Werkstoff (z.B. Holz) eingesetzt werden.

In Figur 10 ist eine Anwendung des vorgeschlagenen Verfahrens im Zusammenhang mit Wirbelsäulenknochen 39 dargestellt. Hier wird in eine ameliorierte Ausnehmung 40 (erzeugt in einem analogen Verfahren wie in den Figuren 1 bis 7 dargestellt) eine Schraube 38 anstelle eines Dentalimplantates eingesetzt. Auch eine solche Schraube 38 verfügt über eine sehr hohe Primärstabilität, eine solche Schraube kann bspw. zur Befestigung einer Relativfixierung 41 verwendet werden.

Der Vollständigkeit halber soll anhand von Figur 11 noch gezeigt werden, wie unterschiedlich die Ameliorationshülse resp. der Führungsstift und die Manschette entsprechend den Bedürfnissen eingestellt werden können. Die normale Situation wird jene sein, wie sie in Figur 11a) dargestellt ist. Hier verfügt die Ameliorationshülse, dargestellt in einem Schnitt senkrecht zur Achse des Führungsstiftes wie mit A-A in Figur 3 dargestellt, über einen Aussendruchmesser D und eine zentrale Ausnehmung mit einem Innendurchmesser d. In diesem Fall handelt es sich um eine relativ dünne Ameliorationshülse, das heisst es wird wenig verflüssigbares Material zur Verfügung gestellt. In dieser Ameliorationshülse ist der Führungsstift 3 mit einem Aussendurchmesser d angeordnet.

Soll nun mehr verflüssigbares Material zur Verfügung gestellt werden, kann ein dünnerer Führungsstift 3 und eine Ameliorationshülse mit grösserer Wandstärke verwendet werden, wie dies mit der gestrichelten Linie 42 angedeutet ist.

Alternative Ausgestaltungen der Querschnittsflächen von Führungsstift 3 und Ameliorationshülse 6 sind in den Figuren b) - e) dargestellt. Es ist mit anderen Worten möglich, dass der Führungsstift über einen bspw. quadratischen Querschnitt verfügt, wie dies in Figur 11b) dargestellt ist. Dabei kann es von Vorteil sein, wenn die Ecken des Quadrates beinahe bis zur Peripherie der Ameliorationshülse ragen, das Quadrat kann aber auch eine kleinere Querschnittsfläche aufweisen. Dies ist für den Fall eines Sechseckigen-Querschnitts in Figur 11 c) dargestellt und für eine dünnere Ameliorationshülse und für einen symmetrisch achteckigen Querschnitt in Figur 11d). Es ist auch denkbar, andere Formen für die Querschnittsfläche des Führungsstiftes 3 vorzusehen, so ist bspw. in Figur 11e) eine Situation dargestellt, in welcher der Führungsstift eine Kreuzförmige-Querschnittsfläche aufweist. Solche Formen sind bspw. zum gezielten Eintrag von verflüssigbarem Material in Richtungen, wo bekanntermassen grosse Porosität vorliegt, möglich.

Insbesondere dann, wenn die Manschette 4 an die Sonotrode angekoppelt ist und die Ultraschall-Schwingungsenergie überträgt, kann es problematisch sein, wenn die Wandstärke der Manschette 4 zu gering wird. Die Wandstärke der Manschette 4 sollte in diesen Situationen wenigstens im Bereich von 0.5-0.8 mm liegen. Auf der anderen Seite kann es aber sein, dass z.B. die Porosität der Wandung der Ausnehmung nicht besonders ausgeprägt ist und entsprechend eine Ameliorationshülse mit einer solchen Wandstärke von 0.5-0.8 mm zu viel Material eintragen würde.

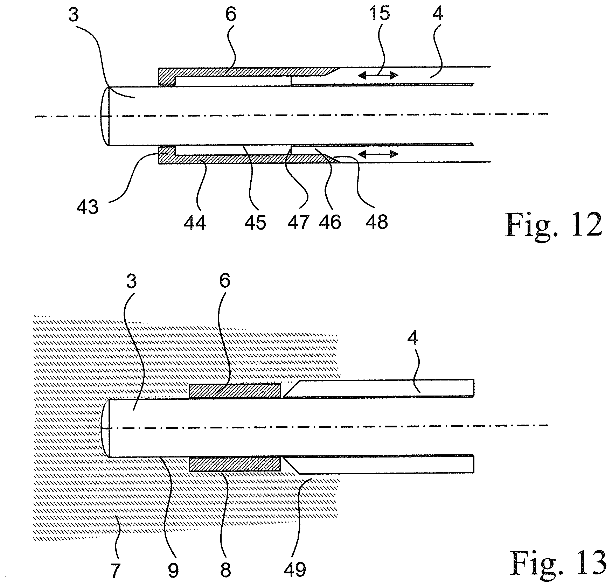

Für eine solche Situation ist das in Figur 12 dargestellte Ausführungsbeispiel von Vorteil. Hier verfügt die Ameliorationshülse 6 über einen weiten axialen Abschnitt über eine geringere Wandstärke als die Manschette 4. Dies wird erreicht, indem unter Belassung eines distalen nach innen gerichteten Flansches 43 (der Flansch kann umlaufend sein, er kann aber auch nur abschnittsweise im Sinne von Vorsprüngen ausgebildet sein) auf der Innenseite die Ameliorationshülse umlaufend ausgenommen (oder auch nicht vollständig umlaufend ausgenommen, das heißt z.B. einige axiale Stege, welche in Kontakt mit dem Führungsstift 3 kommen, belassend). So bildet sich zwischen dem Führungsstift 3 und der nun dünneren Außenwand 44 der Ameliorationshülse ein Hohlraum 45. Diese dünne Außenwand kann eine Wandstärke im Bereich von 0.1-1 mm aufweisen und beispielsweise ohne weiteres nur 0.1-0.3 mm betragen.

Damit nun die Manschette 4 bei einer solchen Ameliorationshülse 6 das Material der Ameliorationshülse trotzdem optimal radial in die Wandung der Ausnehmung eintragen kann, ist die Manschette 4 an ihrem distalen Ende in einem verjüngten zylindrischen Bereich 46 mit einem geringeren Außendurchmesser ausgestaltet. Der radiale Versatz dieser Stufe am distalen Ende der Manschette 4 entspricht dabei im wesentlichen der Wandstärke der Ameliorationshülse im Bereich 44.

Der verjüngte zylindrische Bereich 46 kann an seinem distalen Ende konisch zulaufend ausgebildet sein, kann aber auch, wie in Figur 12 dargestellt, als rechtwinklige Stufe ausgebildet sein.

Im Übergang vom verjüngten zylindrischen Bereich 46 zum dahinter angeordneten Bereich der Manschette 4 mit den eigentlichen Außendurchmesser der Manschette gibt es einen konisch zulaufenden Bereich 48.

Entsprechend greift der verjüngte zylindrische Bereich 46 in den Hohlraum 45 der Ameliorationshülse 6 mit der Spitze ein. Das proximale Ende der Ameliorationshülse stößt damit gegen die geneigte Flanke 48.

Wird nun, wie durch den Pfeil 15 angedeutet, Ultraschall-Schwingung in longitudinaler Richtung über die Manschette eingetragen, so verflüssigt sich die Ameliorationshülse 6 im wesentlichen nur im Bereich des konischen Übergangs 48. Durch die geneigte Flanke in diesem Bereich 48 wird das verflüssigte Material ausschließlich in diesem Bereich selektiv verflüssigt und radial nach außen in den Umfangsbereich der Ausnehmung und die dort vorhandene Porosität eingetragen. Infolge der Tatsache, dass sich das Material der Ameliorationshülse 6 im wesentlichen nur im Bereich 48 verflüssigt, ist auch die geringe Wandstärke des Bereichs 44 in der Regel unproblematisch, die Ameliorationshülse hat trotz der dort geringen Wandstärke kaum eine Tendenz, unter dem longitudinalen Druck beispielsweise in den Hohlraum 45 einzuknicken.

Wird nun die Manschette sukzessive in Figur 12 im eigentlichen Vorgang nach links verschoben so verflüssigt sich entsprechend nur der dünne Wandungsbereich 44 jeweils nur gerade in der Zone 48 und es wird radial jeweils nur gerade in der Zone 48 das verflüssigte Material nach außen getragen, bis die Spitze 47 auf den Flansch 43 stößt. Hier kann der Prozess entweder abgebrochen werden, oder es erfolgt eine weitergehende Verflüssigung, nun im Bodenbereich mit etwas mehr Material der Ameliorationshülse. Um auch im Bodenbereich das Material radial nach außen zu treiben, kann, wie bereits oben erwähnt, der Bereich 47 ebenfalls konisch ausgebildet sein.

Bei dieser Bauweise ist nun die Manschette 4 nur im vordersten zylindrischen Bereich 46 mit einer geringen Wandstärke ausgebildet, und dieser Bereich 46 wird auch im wesentlichen nur zur Führung eingesetzt, während im dahinter angeordneten Bereich, wo die größeren Kräfte anliegen, eine größere Wandstärke eingesetzt werden kann, ohne dass dies auch eine entsprechende Wandstärke der Ameliorationshülse 6 bedingt.

Typischerweise verfügt der vorderste zylindrischer Bereich 46 für Anwendungen im Dentalbereich über eine Länge von circa 0.1-5mm, bevorzugt von 0.1-1mm oder 0.2-0.5mm in axialer Richtung, vorzugsweise eine Länge von 0.1-0.25 mm.

Wie bereits weiter oben erläutert, ist es auch möglich, dass der Außendurchmesser der Manschette 4 etwas größer ist als der ursprüngliche Innendurchmesser der Ausnehmung. Dies wird beispielhaft in Figur 13 dargestellt. Hier ist die Ausnehmung im Bodenbereich 9 wiederum verjüngt, in diesen Bodenbereich ragt der Führungsstift 3 mit seinem distalen Ende hinein. Der Innendurchmesser der Ausnehmung weiter rechts in Figur 13 oberhalb von diesem Bodenbereich entspricht im wesentlichen dem Außendurchmesser der Ameliorationshülse 6, das heißt Stift 3 und Ameliorationshülse 6 können im wesentlichen widerstandslos in die Ausnehmung eingeschoben werden. Die Manschette 4 hingegen verfügt über einen Außendurchmesser, welcher etwas größer ist als der Innendurchmesser der Ausnehmung (der Durchmesser ist beispielsweise 0.1-0.2 mm größer als der Innendurchmesser). Wird nun die Manschette 4 in die Ausnehmung hineingedrückt, so führt dies zu einer erzwungenen Aufweitung der Ausnehmung, wie dies schematisch durch den bereits erweiterten Abschnitt 49 dargestellt ist.

Eine solche erzwungene Aufweitung durch die Manschette 4 kann beispielsweise dann von Vorteil sein, wenn beispielsweise die Ausnehmung nicht die gewünschte Form und/oder den gewünschten Durchmesser hat und durch diesen Vorgang durch die Manschette 4 gewissermaßen bereinigt werden soll.

### BEZUGSZEICHENLISTE

- 1: Vorrichtung
- 2: Handgriff von 1 mit Sonotrode
- 3: Führungsstift
- 4: Manschette
- 5: Kante von 4
- 6: Ameliorationshülse
- 7: Knochen
- 8: zu ameliorierende Bohrung/Ausnehmung in 7
- 9: Verjüngter Bodenbereich von 8
- 10: konische Spitze von 4
- 11: Hohlräume/Kavitäten in 7
- 12: in 11 verdrängtes Material von 6
- 13: Verschiebungsrichtung von 4 auf 3
- 14: transversale Richtung der Ultraschall-Schwingung
- 15: longitudinale Richtung der Ultraschall-Schwingung
- 16: rotative Ultraschall-Schwingung
- 17: Zahn
- 18: Zahnfleisch
- 19: Lücke
- 20: Bohrer
- 21: Befestigungsflansch für Antrieb an 20
- 22: Bohrerzüge
- 23: Verjüngung an Spitze von 20, Führungsspitze
- 24: Bohrrichtung
- 25: obere Befestigung von 4 an 2, Dreh-, Bajonett-, oder Schnappbefestigung
- 26: zentrale Ausnehmung in 4 für 3
- 27: obere Flanke an 6
- 28: Verdrängungsrichtung von verflüssigtem Material (blaue Pfeile)
- 29: ameliorierte Ausnehmung im Knochen
- 30: Implantat
- 31: Vorrichtung zum Eindrehen des Implantats in ameliorierte Ausnehmung
- 32: Halterung
- 33: Umfangsfläche von 29
- 34: Finger
- 35: Drehmomentschlüssel
- 36: Montagevorrichtung für Implantat
- 37: Montagevorrichtung für Implantat mit Möglichkeit des Einsetzens von Sonotrode
- 38: Schraube
- 39: Wirbelsäulenknochen
- 40: ameliorierte Ausnehmung in 39
- 41: Relativfixierung
- 42: schematische Angabe (gestrichelt) einer Situation für eine dickere Ameliorationshülse
- 43: nach innen gerichteter Flansch von Ameliorationshülse
- 44: dünne Wandung von Ameliorationshülse
- 45: Hohlraum zwischen Führungsstift und Ameliorationshülse
- 46: verjüngter zylindrischer Bereich von Manschette
- 47: distales Ende von 46
- 48: konischer Übergangsbereich zum verjüngten zylindrischen Bereich der Manschette
- 49: bereits erweiterter Abschnitt der Ausnehmung

- D: Aussendurchmesser von Manschette/Ameliorationshülse
- d: Aussendurchmesser von Führungsstift respektive Innendurchmesser von Ausnehmung in Manschette und Innendurchmesser von Ameliorationshülse

## Patentansprüche

1. Vorrichtung (1) zur Amelioration einer Ausnehmung (8), insbesondere einer Ausnehmung in einem porösen, löchrigen und durch die Ausnehmung freigelegte Hohlräume aufweisenden Material, umfassend
ein Element (2) zur Erzeugung oder zur Einkopplung von mechanischer Energie, sowie eine zylindrische Manschette (4) mit zylindrischer Mantelfläche mit einem Aussendurchmesser und mit einer zentralen Ausnehmung (26) zur Aufnahme eines Führungsstiftes (3), einen Führungsstift (3) und eine Ameliorationshülse (6),
wobei der Führungsstift (3) vor dem Anlegen von mechanischer Energie im wesentlichen bis auf den Boden der Ausnehmung (8) einführbar ausgestaltet ist, wobei der Führungsstift (3) im Bereich seines dem Boden der Ausnehmung zugewandten Endes von einer Ameliorationshülse (6) aus einem mit mechanischer Energie verflüssigbaren Material umschlossen wird, wobei die zylindrische Mantelfläche der Ameliorationshülse (6) im wesentlichen den gleichen Aussendurchmesser aufweist wie die Manschette (4), wobei der Führungsstift (3) in der zentralen Ausnehmung (26) derart verschieblich aufgenommen ist, dass die Manschette (4) bei Anlegen von mechanischer Energie relativ zum Führungsstift (3) in Richtung zum Boden der Ausnehmung (8) unter Verflüssigung und seitlicher und/oder longitudinaler Verdrängung des Materials der Ameliorationshülse (6) verschiebbar ist,
**dadurch gekennzeichnet, dass**
der Führungsstift (3) bis zu einer Anschlagsposition, bei welcher der Führungsstift (3) bündig mit dem distalen Ende der Manschette (4) abschliesst, in die Manschette (4) einschiebbar ist.

2. Vorrichtung nach Anspruch 1, **dadurch gekennzeichnet, dass** die zylindrische Manschette (4) eine kreiszylindrische Mantelfläche aufweist und die Ameliorationshülse (6) eine kreiszylindrische Mantelfläche aufweist.

3. Vorrichtung nach einem der vorhergehenden Ansprüche, **dadurch gekennzeichnet, dass** die zentrale Ausnehmung (26) eine kreiszylindrische Ausnehmung ist, welche koaxial zur zylindrischen Mantelfläche angeordnet ist, dass die Ameliorationshülse (6) eine kreiszylindrische Ausnehmung zur Aufnahme des Führungsstifts (3) aufweist, und dass der Führungsstift (3) eine kreiszylindrische Aussenfläche aufweist, wobei die Innendurchmesser der genannten Ausnehmungen im wesentlichen gleich sind wie der Aussendurchmesser des Führungsstiftes (3).

4. Vorrichtung nach einem der vorhergehenden Ansprüche, **dadurch gekennzeichnet, dass** die Manschette (4) an ihrem distalen Ende eine zu diesem hin zulaufende bevorzugt umlaufende distale Kante (5, 48) aufweist, wobei vorzugsweise diese Kante (5, 48) gerade und damit konisch oder gekrümmt, insbesondere konkav oder konvex, ausgebildet ist, wobei vorzugsweise die Manschette (4) am distalen Ende radial gestuft ausgebildet ist und die umlaufende distale Kante (48) vorzugsweise beim Stufenübergang angeordnet ist.

5. Vorrichtung nach einem der vorhergehenden Ansprüche, **dadurch gekennzeichnet, dass** der Aussendurchmesser der Manschette (4) im Bereich von 1-80 mm, bevorzugt im Bereich von 2-10 mm beträgt, und dass der Aussendurchmesser des Führungsstiftes (3) 0.1 - 20 mm, bevorzugt 0.1-2 mm oder 0.5-1 mm geringer ist, und dass die Ameliorationshülse eine Dicke aufweist, so dass deren Aussendurchmesser gleich ist wie der Aussendurchmesser der Manschette (4).

6. Vorrichtung nach Anspruch 5, **dadurch gekennzeichnet, dass** die Ameliorationshülse wenigstens abschnittsweise eine Wandstärke im Bereich von 0.1-1mm aufweist.

7. Vorrichtung nach einem der vorhergehenden Ansprüche, **dadurch gekennzeichnet, dass** das Element (2) mechanische Energie in Form von Vibrationsenergie und/oder Schwingungsenergie mit Frequenzen im Bereich von 1 kHz - 10 GHz, bevorzugt in Form von Ultraschall-Schwingungen im Frequenzbereich von 10 kHz - 100 MHz oder 20-150 kHz, besonders bevorzugtermassen im Bereich von 25-50 kHz, erzeugt, welche in longitudinaler, transversaler oder rotativer Richtung, oder einer Kombination respektive Linearkombination dieser Richtungen, bevorzugt im wesentlichen ausschliesslich in longitudinaler Richtung, auf Manschette (4) und/oder Führungsstift (3) und damit mittelbar auf die Ameliorationshülse (6) übertragen werden, wobei bevorzugtermassen die Manschette (4) an der Sonotrode befestigt ist und der Führungsstift (3) in dieser verschoben werden kann, oder der Führungsstift (3) an der Sonotrode befestigt ist und die Manschette (4) verschoben werden kann, oder Manschette (4) und Führungsstift (3) an einer Sonotrode befestigt respektive an solche angekoppelt sind.

8. Vorrichtung nach einem der vorhergehenden Ansprüche, **dadurch gekennzeichnet, dass** die Vorrichtung zur Amelioration einer Ausnehmung (8) in einem wenigstens teilweise porösen technischen Material wie Holz oder holzartigen Material oder Schaumstoffmaterial, insbesondere ein Polymerschaum, ein Verbundschaum und/oder ein Metallschaum, oder einem wenigstens teilweise porösen tierischen oder menschlichen Knochenabschnitt geeignet ist, insbesondere in einem Kieferknochen oder einem Wirbelsäulenknochen, wobei bevorzugtermassen die Ausnehmung wenigstens teilweise durch eine Vorbohrung erzeugt ist.

9. Vorrichtung nach einem der vorhergehenden Ansprüche, **dadurch gekennzeichnet, dass** die Ameliorationshülse (6) aus einem durch die mechanische Energie, insbesondere durch Schwingungsenergie verflüssigbaren Material ausgewählt aus der Gruppe: thermoplastischer biokompatibler Polymere wie Polyolefine ausgewählt aus PP, LDPE, HDPE, UHMWPE, Polyoxymethylen, Polyaryletherketone wie PAEK, PEEK, PEKK, Polycarbonate, Polyacrylate wie PMMA, Polyamide , Polyester wie PET, PBT, Polysulfone und Polyethersulfone wie PSU, PES und/oder biodegradierbare respektive resorbierbare Polymere wie Poly-(L-lactide) (PLLA), poly-(D,L-lactide) (PDLLA), und/oder derer Stereocopolymere mit variierendem Verhältnis des L und D,L Anteils, Polyglycolide (PGA) und/oder Copolymere wie Polyglycolide-co-trimethylene carbonat (PGA-co-TMC), Poly-(D,L-lactide-co-glycolide) (PDLLA-co-PGA), und Poly-(L-lactide-co-glycolide) (PLLA-co-PGA), Poly(e-caprolactone), Polydioxanone, Trimethylene carbonate (TMC), Polyorthoester (POE) und andere Polyanhydride, resorbierbare Polymere welche aus natürlichen Rohstoffen hergestellt werden wie modifizierte Polysaccharide (Zellulose, Chitin, Dextran, Stärke) oder einer Kombination respektive eine Mischung dieser Materialien, wobei bevorzugtermaßen in diesem Material respektive dieser Materialmischung oder als Schicht auf diesem Material aufgetragen auch eine oder mehrere pharmazeutische Wirksubstanzen vorgesehen sein können, wobei diese pharmazeutischen Wirksubstanzen bevorzugtermassen kontrolliert freigesetzt werden.

10. Nicht in vivo Verfahren zum Betrieb einer Vorrichtung nach einem der Ansprüche 1-9, **dadurch gekennzeichnet, dass** die Vorrichtung (1) mit aufgesetztem Führungsstift (3) und aufgesetzter Ameliorationshülse (6) in eine ggf. zuvor ausgebohrte Ausnehmung (8) mit einem Innendurchmesser, welcher im wesentlichen dem Aussendurchmesser von Manschette (4) respektive Ameliorationshülse (6) entspricht, derart eingeschoben wird, bis der Führungsstift (3) auf dem Boden der Ausnehmung (8) anschlägt, und/oder in eine am Boden der Ausnehmung (8) angeordnete Führungsverjüngung (9) eingreift, und anschliessend unter gleichzeitiger Verflüssigung der Ameliorationshülse (6) durch angelegte mechanische Energie, bevorzugtermassen durch angelegten Ultraschall, und Hineinschieben der Manschette (4) mit deren distalem Ende in die Ausnehmung (8) hinein verflüssigtes Material in insbesondere laterale, an die Ausnehmung (8) angrenzende Hohlräume (11) eingetragen wird.

11. Nicht in vivo Verfahren zur Amelioration einer Ausnehmung (8) in einem porösen, löchrigen und durch die Ausnehmung freigelegte Hohlräume aufweisenden Material wie insbesondere einem Schaumstoff, Holz oder einem holzartigen Werkstoff, unter Verwendung einer Vorrichtung nach einem der Ansprüche 1 - 9, **dadurch gekennzeichnet, dass** die Vorrichtung mit aufgesetztem Führungsstift (3) und aufgesetzter Ameliorationshülse (6) in die ggf. zuvor ausgebohrte Ausnehmung (8) mit einem Innendurchmesser, welche im wesentlichen dem Aussendurchmesser von Manschette (4) respektive Ameliorationshülse (6) entspricht, derart eingeschoben wird, bis der Führungsstift (3) auf dem Boden der Ausnehmung (8) anschlägt, und/oder in einer am Boden der Ausnehmung (8) angeordnete Führungsverjüngung (9) der Ausnehmung (8) eingreift, und anschliessend unter gleichzeitiger Verflüssigung der Ameliorationshülse (6) durch angelegte mechanische Energie, insbesondere durch angelegten Ultraschall, und hineinschieben der Manschette (4) mit deren distalem Ende in die Ausnehmung (8) hinein verflüssigtes Material in insbesondere laterale, an die Ausnehmung angrenzende Hohlräume (11) eingetragen wird, wobei bevorzugtermassen anschliessend in die ameliorierte Ausnehmung (29) eine Befestigungsvorrichtung oder eine Schraube, ggf. teilweise selbstschneidend, eingedreht wird.

## Claims

1. A device (1) for the amelioration of a recess (8), particularly of a recess in a porous, perforate material having cavities freed by the recess, said device (1) comprising
an element (2) for generating or coupling in mechanical energy, and a cylindrical collar (4) with cylindrical jacket surface having an external diameter and having a central recess (26) for receiving a guide pin (3), a guide pin (3), and an amelioration sleeve (6)
wherein the guide pin (3) is designed to be insertable substantially as far as the bottom of the recess (8) before mechanical energy is applied, wherein the guide pin (3), in the area of the end thereof directed toward the bottom of the recess, is surrounded by the amelioration sleeve (6) made from a material that can be liquefied by mechanical energy, wherein the cylindrical jacket surface of the amelioration sleeve (6) has substantially the same external diameter as the collar (4), and wherein the guide pin (3) is received movably in the central recess (26) such that, when mechanical energy is applied, the collar (4) being movable relative to the guide pin (3) in the direction toward the bottom of the recess (8) while liquefying and laterally and/or longitudinally displacing the material of the amelioration sleeve (6)
**characterized in that**
the guide pin (3) can be pushed into the collar (4) as far as an abutment position, wherein the guide pin (3), in this abutment position, ends flush with the distal end of the collar (4).

2. The device as claimed in claim 1, **characterized in that** the cylindrical collar (4) has a circular cylindrical jacket surface, and the amelioration sleeve (6) has a circular cylindrical jacket surface.

3. The device as claimed in one of the preceding claims, **characterized in that** the central recess (26) is a circular cylindrical recess which is arranged coaxially with respect to the cylindrical jacket surface, **in that** the amelioration sleeve (6) has a circular cylindrical recess for receiving the guide pin (3), and **in that** the guide pin (3) has a circular cylindrical outer surface, wherein the internal diameters of said recesses are substantially the same as the external diameter of the guide pin (3).

4. The device as claimed in one of the preceding claims, **characterized in that** the collar (4), at its distal end, has a preferably circumferential distal edge (5, 48) tapering toward said distal end, wherein this edge (5, 48) is preferably straight, and therefore conical, or curved, in particular concave or convex, and if the collar (4) has a radially stepped design at its distal end such a circumferential distal edge (48) is arranged at the step transition.

5. The device as claimed in one of the preceding claims, **characterized in that** the external diameter of the collar (4) is in the range of 1-80 mm, preferably in the range of 2-10 mm, and **in that** the external diameter of the guide pin (3) is 0.1-20 mm less, preferably 0.1-2 mm or 0.5-1 mm less, and **in that** the amelioration sleeve has a thickness such that the external diameter thereof is the same as the external diameter of the collar (4) .

6. The device as claimed in claim 5, **characterized in that**, wherein the amelioration sleeve, at least in some sections, has a wall thickness in the range of 0.1-1 mm.

7. The device as claimed in one of the preceding claims, **characterized in that** the element (2) generates mechanical energy in the form of vibration energy and/or oscillation energy with frequencies in the range of 1 kHz - 10 GHz, preferably in the form of ultrasonic oscillations in the frequency range of 10 kHz - 100 MHz or 20-150 kHz, particularly preferably in the range of 25-50 kHz, which are transmitted in the longitudinal, transverse or rotational direction, or in a combination or linear combination of these directions, preferably substantially exclusively in the longitudinal direction, to the collar (4) and/or guide pin (3) and thus indirectly to the amelioration sleeve (6), wherein the collar (4) is preferably secured on the sonotrode, and the guide pin (3) can be moved therein, or the guide pin (3) is secured on the sonotrode, and the collar (4) can be moved, or collar (4) and guide pin (3) are secured on a sonotrode or coupled thereto.

8. Device according to any of the preceding claims, **characterized in that** the device is suitable for the amelioration of recesses (8) in an at least partially porous technical material as wood or wood like material or foam material, in particular a polymer foam, a composite foam and/or a metal foam, or an at least partially porous animal or human bone section, in particular a jawbone or a spinal bone, wherein preferably the recess is generated by a pre-drilling.

9. The device as claimed in one of the preceding claims, **characterized in that** the amelioration sleeve (6) is made from a material that can be liquefied by the mechanical energy, particularly by oscillation energy, and that is selected from the following group: thermoplastic biocompatible polymers such as polyolefins selected from PP, LDPE, HDPE, UHMWPE, polyoxymethylene, polyaryl ether ketones, such as PAEK, PEEK, PEKK, polycarbonates, polyacrylates, such as PMMA, polyamides, polyesters, such as PET, PBT, polysulfones and polyether sulfones, such as PSU, PES and/or biodegradable or resorbable polymers, such as poly(L-lactide) (PLLA), poly(D,L-lactide) (PDLLA) and/or stereocopolymers thereof with a variable ratio of the L and D,L part, polyglycolides (PGA) and/or copolymers, such as polyglycolide-co-trimethyelene carbonate (PGA-co-TMC), poly(D,L-lactide-co-glycolide) (PDLLA-co-PGA) and poly(L-lactide-co-glycolide) (PLLA-co-PGA), poly(e-caprolactone), polydioxanones, trimethylene carbonates (TMC), polyorthoesters (POE) and other polyanhydrides, resorbable polymers which are produced from natural raw materials, such as modified polysaccharides (cellulose, chitin, dextran, starch), or a combination or a mixture of these materials, wherein one or more pharmaceutical active substances can preferably also be provided in this material or this material mixture or applied as a layer on this material, wherein these pharmaceutical active substances are preferably released in a controlled manner.

10. A non-in vivo method for operating a device as claimed in one of claims 1-9, **characterized in that** the device (1), with mounted guide pin (3) and mounted amelioration sleeve (6), is pushed into a recess (8) which has optionally been pre-drilled and which has an internal diameter corresponding substantially to the external diameter of collar (4) and amelioration sleeve (6), until the guide pin (3) abuts against the bottom of the recess (8) and/or engages in a guide taper (9) arranged at the bottom of the recess (8), and then, with simultaneous liquefying of the amelioration sleeve (6) by applied mechanical energy, preferably by applied ultrasound, and with pushing of the distal end of the collar (4) into the recess (8), liquefied material is introduced into cavities (11), particularly lateral cavities (11), adjoining the recess (8).

11. A non-chirurgical method for the amelioration of a recess (8) in a porous, perforate material having cavities freed by the recess, particularly in a foam, wood or a wood-like material, or a human or animal bone (7), using a device as claimed in one of claims 1 - 9, **characterized in that** the device, with mounted guide pin (3) and mounted amelioration sleeve (6), is pushed into the recess (8) which has optionally been pre-drilled and which has an internal diameter corresponding substantially to the external diameter of collar (4) and amelioration sleeve (6), until the guide pin (3) abuts against the bottom of the recess (8) and/or engages in a guide taper (9) of the recess (8) arranged at the bottom of the recess (8), and then, with simultaneous liquefying of the amelioration sleeve (6) by applied mechanical energy, particularly by applied ultrasound, and with pushing of the distal end of the collar (4) into the recess (8), liquefied material is introduced into cavities (11), particularly lateral cavities (11), adjoining the recess, wherein a securing device, an implant or a screw, if appropriate with partial self-tapping, is then preferably screwed into the ameliorated recess (29) .

## Revendications

1. Dispositif (1) pour l'amélioration d'un évidement (8), en particulier d'un évidement dans un matériau poreux, comportant de nombreux trous, et présentant des cavités dégagées sous l'effet de l'évidement, comprenant :
un élément (2), pour produire ou appliquer une énergie mécanique, ainsi qu'un manchon cylindrique (4), ayant une surface latérale cylindrique possédant un diamètre extérieur, et un évidement central (26) destiné à loger une broche de guidage (3) ; une broche de guidage (3) et une douille d'amélioration (6),
la broche de guidage (3), avant application d'une énergie mécanique, étant configurée pour l'essentiel de façon à pouvoir être insérée sur le fond de l'évidement (8), la broche de guidage (3) étant, dans la zone de son extrémité dirigée vers le fond de l'évidement, entourée par la douille d'amélioration (6) en un matériau pouvant se liquéfier sous l'effet d'une énergie mécanique, la surface latérale cylindrique de la douille d'amélioration présentant pour l'essentiel le même diamètre extérieur que le manchon (4), et la broche de guidage (3) étant logée dans l'évidement central (26) en coulissement de telle sorte que le manchon (3), lors de l'application d'une énergie mécanique, puisse être déplacé par rapport à la broche de guidage (3) dans la direction allant vers le fond de l'évidement (8) sous l'effet d'une liquéfaction et d'un déplacement latéral et/ou longitudinal du matériau de la douille d'amélioration (6),
**caractérisé en ce que**
la broche de guidage (3) peut être insérée dans le manchon (4) jusqu'à une position de butée, la broche de guidage (3), dans cette position de butée, se terminant en affleurement avec l'extrémité distale du manchon (4) mm.

2. Dispositif selon la revendication 1, **caractérisé en ce que** le manchon cylindrique (4) comprend une surface latérale de cylindre circulaire, et la douille d'amélioration (6) présente une surface latérale de cylindre circulaire.

3. Dispositif selon l'une des revendications précédentes, **caractérisé en ce que** l'évidement central (26) est un évidement en forme de cylindre circulaire, qui est disposé co-axialement par rapport à la surface latérale cylindrique, **en ce que** la douille d'amélioration (6) présente un évidement en forme de cylindre circulaire pour loger la broche de guidage (3), et **en ce que** la broche de guidage (3) présente une surface extérieure de cylindre circulaire, le diamètre extérieur desdits évidements étant pour l'essentiel égal au diamètre extérieur de la broche de guidage (3).

4. Dispositif selon l'une des revendications précédentes, **caractérisé en ce que**
le manchon (4) présente, en son extrémité distale, une arête distale périphérique (5, 48), courant vers cette extrémité, l'arête (5, 48) étant configurée soit droite et donc conique, soit incurvée, en particulier concave ou convexe, et, au cas où le manchon (4) est pourvu des étagements radiaux en son extrémité distale, une telle arête distale périphérique (48) étant disposée au niveau de la transition entre les étagements.

5. Dispositif selon l'une des revendications précédentes, **caractérisé en ce que** le diamètre extérieur du manchon (4) est compris dans la plage de 1-80 mm, de préférence dans la plage de 2-10 mm, et **en ce que** le diamètre extérieur de la broche de guidage (3) est de 0,1-20 mm plus petit, de préférence de 0,1-2 mm ou de 0,5-1 mm plus petit, et **en ce que** la douille d'amélioration présente une épaisseur, de telle sorte que son diamètre extérieur soit égal au diamètre extérieur du manchon (4).

6. Dispositif selon la revendication 5 précédentes, **caractérisé en ce que** la douille d'amélioration présentant de préférence au moins par zones une épaisseur de paroi comprise dans la plage de 0,1-1 mm.

7. Dispositif selon l'une des revendications précédentes, **caractérisé en ce que** l'élément (2) produit une énergie mécanique sous forme d'une énergie de vibration et/ou d'une énergie d'oscillation, présentant des fréquences comprises dans la plage de 1 kHz-10 GHz, de préférence sous forme de vibrations ultrasonores dans la plage de fréquences de 10 kHz-100 MHz ou de 20-150 kHz, d'une manière particulièrement préférée dans la plage de 25-50 kHz, qui, dans la direction longitudinale, transversale ou périphérique, ou dans une combinaison ou une combinaison linéaire de ces directions, de préférence pour l'essentiel exclusivement dans la direction longitudinale, sont transmises au manchon (4) et/ou à la broche de guidage (3) et donc indirectement à la douille d'amélioration (6), le manchon (4) étant de préférence fixé à la sonotrode, et la broche de guidage (3) pouvant coulisser dans cette dernière, ou encore la broche de guidage (3) étant fixée à la sonotrode et le manchon (4) pouvant coulisser, ou encore le manchon (4) et la broche de guidage (3) étant couplés à la sonotrode, chacun y étant fixé.

8. Procédé selon la revendication 9, **caractérisé en ce que**, pour ce qui concerne l'évidement (8), il s'agit d'un évidement dans un matériau technique, au moins partiellement poreux, tel que le bois ou un matériau de type bois ou un matériau mousse, en particulier une mousse polymère, une mousse composite ou une mousse métallique, ou d'un segment d'os humain ou animal, au moins partiellement poreux, en particulier dans un os de mâchoire ou dans un os de colonne vertébrale, et **en ce que** de préférence l'évidement est produit au moins partiellement par alésage d'un avant-trou.

9. Dispositif selon l'une des revendications précédentes, **caractérisé en ce que** la douille d'amélioration (6) est en un matériau pouvant se liquéfier sous l'effet d'une énergie mécanique, en particulier d'une énergie de vibration, choisi dans le groupe consistant en les polymères biocompatibles thermoplastiques, tels que les polyoléfines choisies parmi le PP, le PEBD, le PEHD, le PE à ultra-haute masse moléculaire, le polyoxyméthylène, les polyaryléthercétones telles que le PAEK, le PEEK, le PEKK, les polycarbonates, les polyacrylates tels que le PNMA, les polyamides, les polyesters tels que le PET, le PBT, les polysulfones et les polyéthersulfones tels que le PSU, le PES et les différents polymères biodégradables résorbables tels que les poly(L-lactides) (PLLA), les poly(D,L-lactides) (PDLLA) et/ou leurs stéréocopolymères, présentant différentes proportions entre les parties L et D,L, les polyglycolides (PGA) et/ou les copolymères tels que le polyglycolide-co-carbonate de triméthylène (PGA-co-TMC), le poly-(D,L-lactide-co-glycolide) (PDLLA-co-PGA) et le poly-(L-lactide-co-glycolide) (PLLA-co-PGA), les poly(ε-caprolactones), les polydioxannones, le carbonate de triméthylène (TMC), les polyorthoesters (POE) et d'autres polyanhydrides, les polymères résorbables qui sont fabriqués à partir de matières premières naturelles, tels que les polysaccharides modifiés (cellulose, chitine, dextran, amidon), ou en une combinaison ou un mélange de ces matériaux, un ou plusieurs principes actifs pharmaceutiques pouvant aussi être prévus de préférence dans ce matériau ou dans ce mélange de matériaux ou sous forme d'une couche appliquée sur ce matériau, ces principes actifs pharmaceutiques pouvant de préférence se libérer d'une manière contrôlée.

10. Procédé non in vivo pour l'exploitation d'un dispositif selon l'une des revendications 1-9, **caractérisé en ce que** le dispositif (1), sur lequel sont montées une broche de guidage (3) et une douille d'amélioration (6), est inséré dans un évidement (8), éventuellement alésé au préalable, ayant un diamètre intérieur, lequel pour l'essentiel correspond au diamètre extérieur du manchon (4) ou de la douille d'amélioration (6), jusqu'à ce que la broche de guidage (3) vienne buter sur le fond de l'évidement (8), et/ou vienne entrer en prise dans un rétrécissement de guidage (9), disposé sur le fond de l'évidement (8), puis le matériau liquéfié par liquéfaction de la douille d'amélioration (6) sous l'effet d'une énergie mécanique appliquée, de préférence sous l'effet de l'application d'ultrasons, et d'une insertion simultanée du manchon (4) par son extrémité distale dans l'évidement (8), est introduit dans des cavités (11), en particulier latérales, contiguës à l'évidement (8) .

11. Procédé non chirurgical pour l'amélioration d'un évidement (8) dans un matériau poreux, comportant de nombreux trous et présentant des cavités dégagées par l'évidement, comme en particulier une mousse, du bois ou un matériau de type bois, ou dans un os humain ou animal (7), par utilisation d'un dispositif selon l'une des revendications 1-9, **caractérisé en ce que** le dispositif, sur lequel sont montées la broche de guidage (3) et la douille d'amélioration (6), est inséré par coulissement dans l'évidement (8), éventuellement alésé au préalable, ayant un diamètre intérieur qui pour l'essentiel correspond au diamètre extérieur du manchon (4) ou de la douille d'amélioration (6), jusqu'à ce que la broche de guidage (3) vienne buter sur le fond de l'évidement (8), et/ou vienne entrer en prise dans un rétrécissement de guidage (9), disposé sur le fond de l'évidement (8), de l'évidement (8), puis on introduit le matériau liquéfié par liquéfaction de la douille d'amélioration (6) sous l'effet d'une énergie mécanique appliquée, en particulier sous l'effet de l'application d'ultrasons, et d'une insertion simultanée du manchon (4) par son extrémité distale dans l'évidement (8), dans des cavités en particulier latérales (11), contiguës à l'évidement, un dispositif de fixation, un implant ou une vis, éventuellement partiellement auto-taraudeuse, étant ensuite de préférence introduit par vissage dans l'évidement amélioré (29).
